# EUROPÄISCHE PATENTSCHRIFT

(11) **EP 3 746 167 B1**
(45) Veröffentlichungstag und Bekanntmachung des Hinweises auf die Patenterteilung: **26.07.2023**
(21) Anmeldenummer: 19704221.1
(22) Anmeldetag: 24.01.2019
(51) Int. Cl.: A61M 25/06

(54) **PUNKTIONSSYSTEM**
PUNCTURE SYSTEM
SYSTÈME DE PONCTION

(30) Priorität: 02.02.2018 DE 102018102390
(43) Veröffentlichungstag der Anmeldung: 09.12.2020
(73) Patentinhaber: Ebnet Medical GmbH, 19055 Schwerin (DE)
(72) Erfinder: Ebnet, Jens, 19055 Schwerin (DE)
(74) Vertreter: Kröncke, Rolf
(86) Internationale Anmeldenummer: PCT/EP2019/051751
(87) Internationale Veröffentlichungsnummer: WO 2019/149615

(56) Entgegenhaltungen:
- EP-A2- 2 662 107
- WO-A1-99/37354
- WO-A1-2012/162677
- WO-A1-2017/017095
- WO-A2-03/077982
- WO-A2-2008/092029
- US-A- 4 763 667
- US-A1- 2004 147 877

## Beschreibung

Die Erfindung betrifft ein Punktionssystem mit einem äußeren tubusförmigen Körper, der zum Verweilen in einem Körperteil eines Lebewesens eingerichtet ist, wobei das Punktionssystem wenigstens einen inneren tubusförmigen Körper und eine Punktionsnadel aufweist, wobei der innere tubusförmige Körper durch ein Arbeitslumen des äußeren tubusförmigen Körpers geführt ist und gegenüber dem äußeren tubusförmigen Körper längsverschieblich ist, und die Punktionsnadel durch ein Punktionslumen des inneren tubusförmigen Körpers geführt ist und der innere tubusförmige Körper gegenüber der Punktionsnadel längsverschieblich ist.

WO 2017/017095 A1 beschreibt ein Punktionssystem, wobei das Punktionssystem aus drei gegeneinander verschieblich geführten Elementen besteht. Aus der EP 2 662 107 A2 ist eine transseptale Nadelvorrichtung bekannt. Aus der WO 2012/162677 A1 ist eine medizinische Zugangsvorrichtung bekannt. Aus der US 2004/0147877 A1 ist ein Kathetereinführungssystem bekannt. Aus der WO 2008/092029 A2 ist eine medizinische Zugangsvorrichtung bekannt. Aus der WO 03/077982 A2 ist eine vaskuläre Einführungsvorrichtung mit einem einstellbaren hämostatischen Verschluss bekannt. Aus der US 4,763,677 ist eine gefäßpenetrierende Kathetervorrichtung bekannt. Aus der WO 99/37354 A1 ist ein Kanüleneinführungssystem bekannt.

Derartige Punktionssysteme können zum Beispiel als zentralvenöser Katheter eingesetzt werden, wobei der äußere tubusförmige Körper als Katheterschlauch eingerichtet sein kann. Dieser wird über eine punktierte periphere Vene in den Körper eingeführt und dessen Spitze herznah in einer zentralen Vene positioniert. Über einen zentralvenösen Katheter können so zum Beispiel Flüssigkeiten, Medikamente oder Chemotherapeutika injiziert werden. Zentralvenöse Katheter sind derartig ausgebildet, auch wochenlang im Patienten verbleiben zu können. Der innere tubusförmige Körper ersetzt den beim standardmäßigen Legen eines zentralvenösen Venenkatheters eingesetzten Seldinger-Draht. Der innere tubusförmige Körper wird als Führungskörper für den Katheterschlauch in die punktierte Vene eingebracht, sodass der Katheterschlauch nur noch über den inneren tubusförmigen Körper vorgeschoben werden muss, bis dieser richtig positioniert ist. Das Punktionssystem ist somit drei-schichtig aufgebaut.

Der Erfindung liegt daher die Aufgabe zugrunde, ein solches Punktionssystem weiterzuentwickeln.

Die Aufgabe wird mit einem Punktionssystem mit den Merkmalen des Anspruchs 1 gelöst. Vorteilhafte Ausgestaltungen sind in den Unteransprüchen beschrieben.

Es wird vorgeschlagen, dass das Punktionssystem ein manuell betätigbares Fixierelement aufweist, das durch manuelle Betätigung zumindest in eine Fixierstellung und in eine Lösestellung stellbar ist, wobei in der Fixierstellung die Längsverschieblichkeit
- des inneren tubusförmigen Körpers gegenüber dem äußeren tubusförmigen Körper und/oder
- der Punktionsnadel gegenüber dem inneren tubusförmigen Körper und/oder
- der Punktionsnadel gegenüber dem äußeren tubusförmigen Körper
aufgehoben oder vermindert ist.

Die Erfindung hat den Vorteil, dass das Punktionssystem einfacher und sicherer durch einen Anwender am Patienten appliziert werden kann als bekannte Lösungen. Hierbei dient das Fixierelement dazu, die unterschiedlichen Schichten des Punktionssystems gegeneinander zu fixieren, wobei dadurch die Längsverschieblichkeit der einzelnen Schichten vermindert oder aufgehoben wird. Auf diese Weise werden unerwünschte Bewegungen der Punktionsnadel, des inneren tubusförmigen Körpers und/oder des äußeren tubusförmigen Körpers vermieden. So ist z.B. denkbar, dass die Längsverschieblichkeit des inneren tubusförmigen Körpers gegenüber dem äußeren tubusförmigen Körper vermindert oder aufgehoben ist. Somit wird vermieden, dass bei Punktion eines Lebewesens durch die Punktionsnadel, unerwünschte Bewegungen des inneren und äußeren tubusförmigen Körpers in Richtung des punktierten Körperteils erfolgen. Es ist aber auch möglich, dass die Punktionsnadel gegenüber dem inneren tubusförmigen Körper in eine Fixierstellung stellbar ist, um nach erfolgter Punktion und Einführung des inneren tubusförmigen Körpers in den punktierten Körperteil eine Bewegung dieser Elemente zu vermeiden. Weiterhin ist es möglich, die Punktionsnadel gegenüber dem äußeren tubusförmigen Körper zu fixieren, um somit eine sichere Führung des inneren tubusförmigen Körpers in den punktierten Körperteil zu gewährleisten. Es ist auch denkbar, dass die Punktionsnadel, der innere tubusförmige Körper und der äußere tubusförmige Körper gegeneinander in eine Fixierstellung stellbar sind, um z.B. nach erfolgter Applikation des Punktionssystems an einem Patienten das Punktionssystem gegen unerwünschte Längsbewegungen der einzelnen Schichten zu sichern. Die unterschiedlichen Fixierstellungen des Punktionssystems können dabei vom Anwender haptisch, z.B. durch entsprechende Markierungen am Punktionssystem, unterscheidbar ausgebildet werden.

In Kombination mit dieser Lösung kann das Punktionssystem ein Dichtelement aufweisen, durch das der innere tubusförmige Körper gegenüber dem äußeren tubusförmigen Körper zumindest am patientennahen Ende abgedichtet ist. Durch eine derartige Abdichtung wird sichergestellt, dass kein Austausch von Fluiden zwischen dem inneren tubusförmigen Körper und dem äußeren tubusförmigen Körper stattfinden kann. So kann auch eine unerwünschte Luftansaugung bei der Aspiration vermieden werden.

In Kombination mit den vorherigen Lösungen kann das Punktionssystem am patientennahen Ende einen zum Einführen des äußeren tubusförmigen Körpers in das Körperteil dienenden Dilatationskörper aufweisen, durch den der innere tubusförmige Körper hindurchgeführt ist, wobei der Dilatationskörper in einem spitzen Winkel von weniger als 11 Grad, ausgehend von einem 360°-System, zuläuft. Der Winkel bezieht sich auf das Winkelmaß zwischen zwei außenliegenden Dilatationsflächen des Dilatationskörpers und nicht auf die Mittelachse des Dilatationskörpers.

Vorteilhafterweise kann der Dilatationskörper in einem spitzen Winkel von weniger als 10,5 Grad zulaufen. Dies hat den Vorteil, dass der äußere tubusförmige Körper einfach und ohne zusätzliche Weitung der Punktionsstelle in das punktierte Körperteil eingeführt werden kann, indem der Dilatationskörper über den inneren tubusförmigen Körper in das punktierte Körperteil geschoben wird, wobei die notwendige Dilatation erfolgt und der äußere tubusförmige Körper in das Körperteil eingeschoben werden kann. Dafür ist erforderlich, dass der Dilatationskörper in einem spitzen Winkel von weniger als 11 Grad, insbesondere weniger als 10,5 Grad zuläuft, sodass ein Vorschieben durch die Haut beim Dilatationsvorgang erfolgen kann. Es ist denkbar, dass der Dilatationskörper aus einem Material besteht oder mit einem Material beschichtet ist, das den Reibungswiderstand herabsetzt, um ein Vorschieben des Dilatationskörpers zu erleichtern. Es ist auch denkbar, dass nach erfolgter Einführung des Dilatationskörpers in das punktierte Körperteil dieser aufgeweitet wird und somit die Punktionsstelle derartig aufweitet, dass der äußere tubusförmige Körper in das punktierte Körperteil eingeschoben werden kann.

Gemäß einer vorteilhaften Weiterbildung kann mittels des Fixierelementes in der Fixierstellung die Längsverschieblichkeit auf ein durch die manuelle Betätigung einstellbares Maß verminderbar sein. Vorteilhafterweise kann das Fixierelement durch manuelle Betätigung in unterschiedliche Fixierstellungen stellbar sein, in denen die Längsverschieblichkeit auf jeweilige voneinander unterschiedliche Maße verminderbar ist. Dies hat den Vorteil, dass die Längsverschieblichkeit vom Anwender je nach Bedarfsfall auf unterschiedliche Maße vermindert werden kann. So ist es z.B. möglich, die Längsverschieblichkeit aufzuheben oder nur um ein gewisses Maß zu vermindern, das heißt den Widerstand der einzelnen Schichten gegeneinander zu erhöhen, wenn dies der Anwender für erforderlich hält. Erfindungsgemäß ist das Fixierelement durch manuelle Betätigung in unterschiedliche Fixierstellungen stellbar, in denen die Längsverschieblichkeit hinsichtlich unterschiedlicher Kombinationen der Längsverschieblichkeit
- des inneren tubusförmigen Körpers gegenüber dem äußeren tubusförmigen Körper, ,
- der Punktionsnadel gegenüber dem inneren tubusförmigen Körper, ,
- der Punktionsnadel gegenüber dem äußeren tubusförmigen Körper
einstellbar ist.

In einer vorteilhaften Ausgestaltung kann das Fixierelement des Punktionssystems als Klemmelement ausgebildet sein oder ein Klemmelement aufweisen, wobei mittels des Klemmelementes
- der innere tubusförmige Körper gegenüber dem äußeren tubusförmigen Körper und/oder
- der Punktionsnadel gegenüber dem inneren tubusförmigen Körper und/oder
- der Punktionsnadel gegenüber dem äußeren tubusförmigen Körper
festklemmbar ist.

Durch die Bereitstellung eines Klemmelementes können die einzelnen Schichten des Punktionssystems gegeneinander teilweise oder komplett fixiert werden, um eine sichere Lage des Punktionssystems gewährleisten zu können. Das Klemmelement kann dabei als eine Art Spannzange mit einer kegelförmig geschlitzten Hülse und einer Überwurfmutter ausgebildet sein, wobei durch Anziehen der Überwurfmutter die einzelnen Schichten in den Innenkegel der Hülse gedrückt und damit gespannt werden. Denkbar sind aber auch folgende Alternativen:
- Ein Expansionskörper, der zur Fixierung der einzelnen Schichten in das Punktionssystem eingebracht wird, wobei durch Expansion des Expansionskörpers die innenliegenden Schichten gegen die außenliegenden Schichten gedrückt und damit fixiert werden.
- Ein Bügelsystem, wobei durch Umklappen eines außerhalb des Punktionssystems befindlichen Bügels durch Druck die einzelnen Schichten gegeneinander fixiert werden.
- Eine Schraube, die über eine entsprechende Bohrung in das Punktionssystem eingebracht ist, wobei die Schraube durch Drehen die unterschiedlichen Lagen durch Druck gegeneinander fixiert.
- Eine Beschichtung der einzelnen Schichten des Punktionssystems kann dafür eingerichtet sein, dass die Längsverschieblichkeit im Gegensatz zum herkömmlichen System eingeschränkt ist.
- Es ist auch möglich, dass die einzelnen Schichten z.B. durch Einkerbungen ineinander einrastbar sind.
- Die einzelnen Schichten können dadurch ineinander verkeilbar sein, dass sie einen nicht kreisrunden oder an einzelnen Stellen erhöhten Durchmesser besitzen und somit durch Drehung oder Vor- und Zurückziehen der einzelnen Schichten gegeneinander zumindest eine teilweise Fixierung erfolgt.
- Das Klemmelement kann derartig ausgebildet sein, dass es in einer unberührten Position, z.B. durch einen Federmechanismus, alle Schichten gegeneinander fixiert. Durch Druck oder Zug am Klemmelement wird die Fixierung der Schichten aufgehoben und die Feder gespannt. Erfährt das Klemmelement keinen Druck oder Zug, so kehrt es aufgrund des Federmechanismus in seine Ausgangsposition zurück und fixiert alle Schichten gegeneinander. Diese Ausführung des Klemmelements hat den Vorteil, dass das Klemmelement z.B. mit nur einem Finger bedienbar ist.

Die beschriebenen Funktionen des Fixierelementes sind regel- und wiederholbar, sodass eine große Flexibilität des Fixierelementes gewährleistet ist. Insbesondere soll das Fixierelement auch dafür sorgen, dass das Punktionssystem auf einzelne Lebewesen und deren individuelle Anatomie bzw. auf die Anatomie verschiedener Körperregionen angepasst werden kann. Es kann die vermutete Unterhauttiefe der Struktur, welche punktiert werden soll, bereits vor Beginn des Punktionsvorgangs berücksichtigt werden, indem der aus dem inneren tubusförmigen Körper herausragende spitze Anteil der Punktionsnadel in seiner freien Länge zum zu punktierenden Lebewesen hin limitiert werden kann. Dadurch wird zudem die Gefahr reduziert, dass anatomisch tieferliegende Strukturen nicht versehentlich punktiert werden, insbesondere von einem unerfahrenen Anwender. Dies stellt einen wesentlichen Sicherheitsaspekt des neuen Punktionssystems dar.

In einer vorteilhaften Weiterbildung ist das Dichtelement an der Außenseite und/oder Innenseite des inneren tubusförmigen Körpers fixiert. Ferner vorteilhaft ist es, wenn das Dichtelement als Kunststoffbeschichtung des inneren tubusförmigen Körpers ausgebildet ist, insbesondere als PTFE-Beschichtung. Dies hat den Vorteil, dass auf einfache konstruktive Weise ein Fluidaustausch zwischen dem inneren tubusförmigen Körper und dem äußeren tubusförmigen Körper effektiv und effizient unterbunden wird.

In einer vorteilhaften Weiterbildung weist der innere tubusförmige Körper an der Außenseite eine unebene Oberfläche auf. Eine unebene Oberfläche kann z.B. dadurch entstehen, dass der innere tubusförmige Körper aus einem spiralförmig aufgewickelten Material besteht, wie zum Beispiel bei einer Spiralfeder, wodurch eine unebene Oberfläche erzeugt wird. Durch eine derartige Wicklung kann die Flexibilität des inneren tubusförmigen Körpers erhöht werden, sodass eine erleichterte Applikation am Patienten ermöglicht wird. Das Material kann zudem bevorzugt sonographisch und röntgenologisch gut darstellbar sein. Bei einer unebenen Oberfläche kann es von Vorteil sein, wenn diese zumindest in einem Teilbereich des patientennahen Endes durch das Dichtelement überdeckt ist, insbesondere luftdicht überdeckt ist. Bei der Herstellung eines inneren tubusförmigen Körpers mit einer unebenen Oberfläche kann es dazu kommen, dass insbesondere bei einer spiralförmigen Wicklung der innere tubusförmige Körper gegenüber dem äu-ßeren tubusförmigen Körper Fluiddurchlässig ist. Durch das Dichtelement kann zumindest in einem Teilbereich des patientennahen Endes des Punktionssystems auf einfache Weise ein Fluidaustausch zwischen dem inneren tubusförmigen Körper und dem äußeren tubusförmigen Körper verhindert werden.

Es ist auch möglich, dass ein tubusförmiger Hohlkörper zur Applikation an einem Lebewesen zumindest teilweise aus einem Metall besteht, wobei der tubusförmige Hohlkörper eine unebene Oberfläche hat und an der Außenseite oder an der Innenseite zumindest teilweise mit einer abdichtenden Beschichtung versehen ist, ohne in einem speziellem Punktionssystem integriert zu sein. Ferner vorteilhaft ist es, wenn der tubusförmige Hohlkörper eine spiralförmige Struktur hat. Durch die spiralförmige Struktur hat der tubusförmige Hohlkörper eine große Flexibilität, die eine erleichterte Applikation am Patienten ermöglicht. Eine zumindest teilweise Abdichtung des tubusförmigen Hohlkörpers durch die Beschichtung sorgt dafür, dass die Flexibilität erhalten bleibt und sogleich der tubusförmige Hohlkörper fluidundurchlässig wird, sodass zum Beispiel eine Entnahme von Körperflüssigkeiten oder eine Medikamentengabe durch den tubusförmigen Hohlkörper ermöglicht wird.

Sofern erforderlich, kann der tubusförmige Hohlkörper zusätzlich mit weiteren Strukturen bzw. Elementen verstärkt werden, so z.B. durch quer-, längs- oder diagonal verlaufende Strukturen bzw. Elemente, die auch eine gewisse Strecke des tubusförmigen Hohlkörpers umfassen bzw. überbrücken können und somit einer Entspiralisierung des tubusförmigen Hohlkörpers entgegenwirken.

Eine Abdichtung des tubusförmigen Hohlkörpers kann zum Beispiel über eine Kunststoffbeschichtung wie zum Beispiel einer PTFE-Beschichtung an der Innen-und/oder Außenseite des tubusförmigen Hohlkörpers erfolgen. Der tubusförmige Hohlkörper kann dabei aus einem Metall wie zum Beispiel Edelstahl bestehen. Dadurch kann auch eine elektrische Leitfähigkeit des tubusförmigen Hohlkörpers erreicht werden, sodass zum Beispiel ein EKG-Signal zur Lagekontrolle im Körper abgeleitet werden kann.

Ein tubusförmiger Hohlkörper kann zum Beispiel wie eine Spiralfeder aus einem Metall aufgewickelt werden. Dabei können die einzelnen Schichten eng aneinander liegen, sodass diese miteinander in Kontakt stehen. Dies ähnelt dem Aufbau des bereits bekannten Seldinger Drahtes, der aus einem eng gewundenen Stahldraht besteht. Durch die eng aufgewickelten Schichten aus Metall ist der tubusförmige Hohlkörper schnitt- und stichfest ausgebildet. Eine abdichtende Beschichtung an der Außen- und/oder an der Innenseite des tubusförmigen Hohlkörpers bildet den tubusförmigen Hohlkörper fluidundurchlässig aus. So kann dieser zum Beispiel bei einer Aspiration keine Luft ziehen. Vorteilhafterweise kann die Spitze des tubusförmigen Hohlkörpers am patientennahen Ende zurückbiegbar sein, um eine Verletzung des punktierten Körperteils zu vermeiden.

Denkbar ist aber auch, dass der tubusförmige Hohlkörper über den Umfang verteilt angeordnete Nuten, wie zum Beispiel ein Wellrohr, hat, die eine unebene Oberfläche bereitstellen und somit eine hohe Flexibilität des tubusförmigen Hohlkörpers erreicht wird. Die Nuten verlaufen durchgehend über den kompletten Umfang des tubusförmigen Hohlkörpers und sind vorteilhaft parallel in gleichmäßigen oder ungleichmäßigen Abständen über die Länge des tubusförmigen Hohlkörpers verteilt angeordnet. Der tubusförmige Hohlkörper hat damit einen wechselnden Durchmesser. Auf diese Weise ist es möglich, dass der tubusförmige Körper in verschiedene Positionen ausrichtbar ist. Denkbar ist aber auch, dass sich die Nuten spiralförmig über den Umfang verteilt erstrecken können. Derartige spiralförmige Nuten haben den Vorteil, dass der Druckverlust eines durch den tubusförmigen Hohlkörper geleiteten Fluides verringert wird und gleichzeitig eine Verwirbelung des Fluides erreicht werden kann.

Ein derartiger tubusförmiger Hohlkörper kann in unterschiedlichen medizinischen Anwendungsbereichen zum Einsatz kommen. So ist zum Beispiel der Einsatz als Beatmungsschlauch möglich. Grundsätzlich ist eine Anwendung immer dann möglich, wenn zwei oder mehrere fluidgefüllte Räume miteinander kommunizierend verbunden werden sollen, wobei sich ein oder mehrere Räume davon in einem Lebewesen und ein oder mehrere Räume z.B. in einem technischen Gerät befinden können. Es ist aber auch eine Anwendung zur Pleurapunktion, Blasenpunktion, Luftröhrenpunktion oder Abszesspunktion mit oder ohne folgende Katheteranlage denkbar. Grundsätzlich ist eine Anwendung bei der Punktion und/oder Katheteranlage aller Körperhöhlen und Körperzwischenräume möglich. Dies wird vor allem durch die korrekte Lagebestimmung des tubusförmigen Hohlkörpers ermöglicht, da zu jedem Zeitpunkt eine Aspiration des vermuteten Inhalts der zu punktierenden Struktur möglich sein kann. Liegt der tubusförmige Hohlkörper in einem punktierten Körperteil ein, so kann jederzeit eine Lagebestimmung dadurch erfolgen, dass der tubusförmige Hohlkörper sowohl mit dem Inhalt der zu punktierenden Struktur als auch mit dem Aspirationsmittel kommuniziert, zum Beispiel durch Gasaustausch oder über eine Flüssigkeitssäule.

Es entsteht ein neuartiges Punktionsprinzip dadurch, dass eine Lagebestimmung auf die eben beschriebene Art und Weise beliebig oft erfolgen kann, wenn das Punktionssystem seine endgültige Position noch nicht erreicht hat bzw. eine optimale endgültige Position des Punktionssystems vom Anwender noch gesucht wird.

Der tubusförmige Hohlkörper kann dabei unabhängig von einem Punktionssystem eingesetzt werden. So kann dieser in Kombination mit einem Punktionssystem verwendet werden, dies ist aber keine Voraussetzung für eine Anwendung am Patienten. Der tubusförmige Hohlkörper kann zum Beispiel als innerer tubusförmiger Körper des oben beschriebenen Punktionssystems verwendet werden, um eine große Flexibilität des inneren tubusförmigen Körpers zu erreichen und somit die Applikation am Patienten zu erleichtern. Unter einem Patienten können sowohl menschliche als auch tierische Lebewesen verstanden werden.

In einer vorteilhaften Weiterbildung kann das Punktionssystem wenigstens ein Befestigungselement aufweisen, wobei das Befestigungselement zum Fixieren des Punktionssystems an einem Patienten ausgebildet ist. So kann eine sichere Verwendung des Punktionssystems im laufenden Betrieb sichergestellt werden. Die Fixierung des Punktionssystems kann z.B. durch Festnähen an der Haut erfolgen. Denkbar ist aber auch ein Click-/Clipmechanismus zur Fixierung des Punktionssystems an der Haut. Durch einen Click-/Clipmechanismus werden feine Hautklammern bzw. das Befestigungselement in Richtung der Haut gedrückt, sodass sich die Hautklammern in die Haut bzw. in das Bindegewebe eindringen und für eine sichere Fixierung des Punktionssystems sorgen. Ein Lösen des Punktionssystems kann dabei aber jederzeit erfolgen, indem die Hautklammern einfach aus dem Patienten herausgezogen werden können. Dadurch kann ein zeitaufwendiges Annähen des Punktionssystems entfallen. Zudem kann der eben beschriebene Vorgang des Click-/Clipmechanismus beliebig wiederholt werden. So kann das Punktionssystem bei zu tiefem Einführen in den Körper zur Lagekorrektur eine gewisse Strecke herausgezogen werden. Dadurch ist kein erneutes Festnähen mehr erforderlich.

Denkbar ist auch, dass das Punktionssystem flüssigkeitsgefüllte Elemente aufweist, die z. B. als Gelkissen ausgebildet sein können und eine klebende bzw. haftende Flüssigkeit oder ein Gel enthalten. Bei Berührung reißen die Gelkissen ein, wobei die haftende Flüssigkeit oder das Gel aus den Gelkissen austritt und das Punktionssystem durch die haftende Flüssigkeit oder das Gel an dem Patienten fixiert wird.

Das Punktionssystem kann einen Haltekörper zum erleichterten Halten des Punktionssystems durch den Anwender aufweisen. Durch einen derartigen Haltekörper ist das Handling des Punktionssystems für den Anwender verbessert, sodass eine einfachere Applikation am Patienten erreicht werden kann. Ferner vorteilhaft ist es, wenn der Haltekörper eine Ausnehmung hat, wobei die Ausnehmung zum Führen eines Fadens eingerichtet ist. Dabei kann durch eine Annaht ein Zug in Richtung des Patienten ausgeübt werden, sodass die Fixierung des Punktionssystems verbessert werden kann. Ein zweiter Haltekörper kann am patientennahen Ende angeordnet sein. Dieser Haltekörper kann so gestaltet sein, dass er fest, aber gegen die äußerste Schicht des Punktionssystems verschieblich anordbar ist. Dadurch kann das Punktionssystem sicher mit zwei Händen bedient werden.

Gemäß einer vorteilhaften Weiterbildung hat das Punktionssystem einen Mandrin zur Stabilisierung des inneren tubusförmigen Körpers, des äußeren tubusförmigen Körpers und/oder der Punktionsnadel. Ein Mandrin dient als Hilfsmittel zur Einführung der tubusförmigen Körper und/oder der Punktionsnadel in das punktierte Körperteil. Der Mandrin kann nach Positionierung zumindest einer Schicht des Punktionssystems entfernt werden. Zudem kann der Mandrin einen Betrag zurückgezogen werden, sofern dies erforderlich ist. Ein Mandrin kann z.B. als hohle Kanüle ausgebildet sein, wobei der Mandrin das Führen der einzelnen Schichten stabilisiert und damit erleichtert.

Zudem kann durch einen Mandrin verhindert werden, dass durch die Punktionsnadel ein Hautzylinder entfernt bzw. ausgestanzt wird, der während des weiteren Einführvorgangs tiefer in den Körper gelangen kann. Damit werden neue Anwendungsgebiete für das Punktionssystem wie zum Beispiel rückenmarknahe und allgemein nervennahe Anwendungen erschlossen. Dabei kann es von Vorteil sein, wenn der Mandrin nicht hohl, sondern massiv ausgebildet ist. Der Mandrin füllt dabei das Lumen eines tubusförmigen Körpers komplett aus, um keinen Gewebezylinder auszustanzen.

In der Punktionsnadel kann sich ein Mandrin befinden, der entweder von dem Fixierelement in seiner Position ebenso verstellbar und in dieser fixierbar sein oder von der Funktion des Fixierelements unberührt bleiben kann.

Vorteilhaft ist es, wenn der innere tubusförmige Körper zumindest teilweise aus einem Metall besteht oder mit einem Metall beschichtet ist. Auf diese Weise kann ein versehentliches Durchstechen des inneren tubusförmigen Körpers durch die Punktionsnadel verhindert werden.

Es ist vorteilhaft, wenn nach erfolgter Punktion eines Körperteils mittels eines aus einem patientennahen Ende des Punktionssystems herausragenden Punktionsabschnittes der Punktionsnadel der innere tubusförmige Körper aus dem patientennahen Ende des Punktionssystems zumindest teilweise aus dem äußeren tubusförmigen Körper herausschiebbar und dabei aus dem patientennahen Ende des Punktionssystems herausragenden Punktionsabschnitt der Punktionsnadel wenigstens zum Teil in dem Punktionslumen des inneren tubusförmigen Körpers aufnehmend ausgebildet ist. Dies hat den Vorteil, dass der äußere tubusförmige Körper wesentlich einfacher und schneller am Patienten appliziert werden kann. Durch den mehrschichtigen Aufbau kann der innere tubusförmige Körper nach erfolgter Punktion über die Punktionsnadel in die so gebildete Öffnung eingeschoben werden. Dabei schützt der innere tubusförmige Körper bereits das punktierte Gefäß vor Verletzungen durch die Spitze der Punktionsnadel. Der innere tubusförmige Körper fungiert als Einführhilfe des äußeren tubusförmigen Körpers, indem der äußere tubusförmige Körper an die gewünschte Position über den inneren tubusförmigen Körper geschoben werden kann.

Der innere tubusförmige Körper kann aber auch in seiner Position belassen werden, wenn sich das Punktionssystem in der endgültigen Position befindet. Dies kann insbesondere auch dann sinnvoll sein, wenn weitere sich im Körperinneren befindende Strukturen, wie zum Beispiel kleine Blutgefäße, selektiv durch die patientennahe Öffnung des inneren tubusförmigen Körpers erreicht werden sollen. Durch eine röntgendichte Ausgestaltung des inneren tubusförmigen Körpers kann seine Position zusätzlich verifiziert werden. Außerdem kann zum Beispiel Kontrastmittel durch den inneren tubusförmigen Körper injiziert werden. Durch die genannten Eigenschaften können insbesondere auch Anwendungsbereiche der interventionellen Medizin erschlossen werden.

Das Punktionssystem kann am patientenfernen Ende ein Aspirationshilfsmittel oder einen Aspirationsanschluss zum Anschluss eines Aspirationshilfsmittels aufweisen. Das Aspirationshilfsmittel, das z.B. als konventionelle Spritze ausgebildet sein kann, kann über den Aspirationsanschluss z.B. mit der Punktionsnadel verbunden werden. Es ist aber auch denkbar, dass das Aspirationshilfsmittel mit dem inneren oder äußeren tubusförmigen Körper verbunden werden kann. Hierdurch kann zu jedem Zeitpunkt durch Aspiration kontrolliert werden, ob sich der patientennahe Anteil des Punktionssystems noch in der Zielstruktur befindet. So kann zum Beispiel durch Aspiration von Blut verifiziert werden, ob sich der patientennahe Anteil des Punktionssystems noch in einem Blutgefäß befindet.

An dem Punktionssystem kann ein Unterdruckelement angeordnet sein, wobei nach erfolgter Punktion sich die Unterdruckkammer automatisch mit Flüssigkeit bzw. Blut füllt. Dadurch kann die Lage des Punktionssystems auf einfache Weise ermittelt werden, da je nach Farbe des Blutes sich das Punktionssystem in einem venösen oder arteriellen Gefäß befindet. Das Unterdruckelement kann zum Beispiel anstelle eines Aspirationsmittels angeschlossen werden. Alternativ kann auch ein Unterdruckelement an einen Schenkel oder einem Dreiwegehahn des Punktionssystems angeschlossen werden. Alternativ oder zusätzlich können auch mehrere Unterdruckelemente auf die eben genannten Arten angeschlossen werden. Diese sind einfach anbringbar und auch wieder entfernbar.

Das Punktionssystem kann eine aufblasbare Manschette, einen sogenannten Cuff, haben, der z.B. an der Außenseite des äußeren tubusförmigen Körpers angeordnet ist. Durch einen derartigen Cuff kann eine Abdichtung des Punktionssystems nach außen erfolgen, indem der unter die Haut eingebrachte Cuff aufgeblasen oder mit Flüssigkeit gefüllt wird und das Punktionssystem nach außen somit abgedichtet wird. Zusätzlich kann das Punktionssystem, insbesondere in Hohlkörpern wie z.B. der Luftröhre, dadurch auch in seiner Lage stabilisiert werden.

Das Punktionssystem kann am patientenfernen Ende ein zwischengeschaltetes Y-Stück haben, wobei an dem Y-Stück Dreiwegehähne montiert werden können. Die Dreiwegehähne können dabei an einem Fortsatz des Y-Stückes angeschlossen werden. Es ist aber auch denkbar, dass die Dreiwegehähne direkt an das Y-Stück angeschlossen werden können. Dies könnte zum Beispiel im Zuge eines Rastelementes am Y-Stück erfolgen, indem ein Dreiwegehahn in ein Rastelement gesteckt werden kann. Das Y-Stück kann dabei um seine Längsachse drehbar gelagert sein, sodass das Y-Stück durch den Anwender in eine vorteilhafte Position gedreht werden kann. Eine separate Lagerung der Dreiwegehähne ist dabei auch möglich, sodass eine sehr hohe Flexibilität gewährleistet ist und dem Anwender eine optimale Bedienung ermöglicht. Die Dreiwegehähne können dabei zum Beispiel um Ihre eigene Achse drehbar gelagert sein. Vorteilhafterweise können auch Filter in den Dreiwegehähnen integriert sein, die zum Beispiel das Eindringen von Luft, Partikeln oder Bakterien in den Körper eines Lebewesens verhindern können. Alternativ können derartige Filter aber auch an anderen Positionen des Punktionssystems integriert werden.

Das Punktionssystem kann dabei modular aufgebaut sein, sodass Teile des Punktionssystems entfernt werden können, wenn diese nicht benötigt werden. So kann zum Beispiel das Y-Stück abmontiert werden, wenn keine Teilung des Katheterschlauchs notwendig ist, zum Beispiel keine Teilung in mehrere Lumen, Arbeits-oder Durchflusskanäle. Dies könnte zum Beispiel über eine Schraubverbindung erfolgen. Das Y-Stück kann dabei als abnehmbare Abdeckplatte ausgestaltet sein.

Das Punktionssystem kann vorteilhaft zumindest teilweise transparent ausgestaltet sein, um Fluide in dem Punktionssystem erkennen und identifizieren zu können. So kann auch die Flussrichtung des Fluides bestimmt werden.

Eine weitere Besonderheit des erfindungsgemäßen Katheters besteht darin, dass ein kleinlumiger innerer tubusförmiger Körper verwendet werden kann. Dies hat den Vorteil, dass die durch die Punktionsnadel geschaffene Hautöffnung beim Durchschieben des inneren tubusförmigen Körpers nicht wesentlich geweitet werden muss. Insbesondere ist hierbei keine Dilatation im üblichen medizinischen Sinne erforderlich. Daher kann der erfindungsgemäße Katheter auch ohne Dilatationskanüle realisiert werden. Stattdessen hat der innere tubusförmige Körper die Funktion einer Sicherung des durch die Punktionsnadel geschaffenen Lumens. Der innere tubusförmige Körper kann daher auch als Lumensicherungskanüle bezeichnet werden. Gemäß einer vorteilhaften Weiterbildung der Erfindung ist der Außendurchmesser des inneren tubusförmigen Körpers daher maximal doppelt so groß wie der Außendurchmesser der Punktionsnadel. In einer vorteilhaften Weiterbildung ist der Außendurchmesser des inneren tubusförmigen Körpers maximal 1 ,5-mal so groß ist wie der Außendurchmesser der Punktionsnadel. Die Durchmesser können dabei Anwendungsspezifisch ausgestaltet sein. So sind zum Beispiel sowohl große als auch kleinere Durchmesser des Punktionssystems denkbar.

Im Unterschied zu anderen, bekannten Vorschlägen wird somit eine Punktionsnadel vorgeschlagen, die einen geringeren Durchmesser hat als der Katheterschlauch und somit nur ein kleines Loch in der Haut hinterlässt.

Hierin liegt auch ein entscheidender Vorteil gegenüber bekannten Katheter-Lösungen. Bei bekannten Systemen wird z.B. eine Kanüle mit einem großen Durchmesser verwendet, durch die dann der Katheterschlauch in die Vene eingeführt wird. Die Kanüle kann belassen oder ggf. gespalten und entfernt werden. Das Punktionsloch in der Haut weist bei solchen Systemen einen größeren Durchmesser als der Katheterschlauch auf, wodurch es in der Regel zu Blutungen aus der Hauteintrittsstelle des Katheterschlauchs kommt. Zudem wird der Durchmesser des einzuführenden Katheterschlauchs durch den Durchmesser der Kanüle begrenzt. Da diese bereits einen gewissen Durchmesser haben muss, ist der Durchmesser des Katheterschlauchs begrenzt, so dass keine besonders hohen Flüssigkeitsmengen pro Zeiteinheit in die Vene infundiert werden können. Nachteilig bei bekannten Systeme ist ferner, dass die zur Punktion verwendete Punktionsnadel einen ebenso großen Durchmesser hat, so dass die Punktion traumatisierend ist. Fehlpunktionen können erhebliche Verletzungen zur Folge haben. Solche Nachteile werden durch die vorliegende Erfindung überwunden.

Dementsprechend ist im Übergang von der Punktionsnadel zum inneren tubusförmigen Körper nur ein geringer Kalibersprung vorhanden. Um das Einführen des inneren tubusförmigen Körpers noch weiter zu vereinfachen, kann diese am patientennahen Ende abgerundet ausgebildet sein.

Hinsichtlich des äußeren tubusförmigen Körpers ergibt sich im Vergleich zum inneren tubusförmigen Körper ein größerer Kalibersprung, was notwendig ist, um auch einen erforderlichen Innendurchmesser zu realisieren, der für die Zuführung von Flüssigkeiten in hoher Menge notwendig ist. Gemäß einer vorteilhaften Weiterbildung der Erfindung ist daher der Außendurchmesser des äußeren tubusförmigen Körpers wenigstens doppelt so groß wie der Außendurchmesser des inneren tubusförmigen Körpers. Hierdurch werden große Durchflussmengen gewährleistet. Gemäß einer vorteilhaften Weiterbildung der Erfindung ist der Außendurchmesser des inneren tubusförmigen Körpers wenigstens dreimal so groß wie der Außendurchmesser des inneren tubusförmigen Körpers.

Dementsprechend ist erst beim Einführen des äußeren tubusförmigen Körpers in die Vene ein Dilatationsschritt erforderlich.

Das Punktionssystem kann eine sterile Hülle haben, wobei die sterile Hülle als vierte Schicht über dem äußeren tubusförmigen Körpers bzw. Dem Punktionssystem liegen kann. Durch eine derartige sterile Hülle kann das Punktionssystem zusätzlich stabilisiert werden.

Die sterile Hülle, insbesondere am patientennahen Ende zugewandte Hülle, kann aus festerem Material ausgebildet sein. Dadurch wird sie zwar beim Vorschieben des Punktionssystems, insbesondere des äußeren tubusförmigen Körpers, an der Haut zurückgehalten, stabilisiert aber zusätzlich das Punktionssystem, insbesondere dann, wenn sich der äußere tubusförmige Körper außerhalb des Körpers befindet. Dadurch, dass die sterile Hülle die Haut nicht passieren kann, entsteht ein Punktionssystem, welches außerhalb des Körpers eher feste Eigenschaften aufweist, innerhalb des Körpers aber eher flexible Eigenschaften besitzt.

Denkbar wären auch saugnapfähnliche Elemente, die zu Beginn der Applikation stabilisierend auf die Haut aufgesetzt werden können und so ein Verrutschen des Punktionssystems verhindern. Es ist aber auch ein stempelförmiger Aufsatz möglich, der vor der Applikation stabilisierend auf die Haut aufgesetzt werden kann. Möglich ist zum Beispiel eine wulstartige oder andersartig ausgebildete Aufweitung des patientennahen Endes der sterilen Hülle, zum Beispiel in Form eines Schutzringes, der auch separat montiert werden kann. Alternativ kann die Aufweitung auch kappen-, vorsprungs-, klappenartig ausgebildet sein.

Zwischen der sterilen Hülle und dem äußeren tubusförmigen Körper kann wenigstens ein Gleitelement angeordnet sein, wodurch eine optimale Längsverschieblichkeit der beiden Schichten gewährleistet ist. Das Gleitelement kann zum Beispiel als Kugel oder als Fläche mit kugelähnlichen Unterelementen ausgebildet sein, wobei die Kugel den Reibungswiderstand des äußeren tubusförmigen Körpers gegenüber der sterilen Hülle herabsetzt.

Zudem kann alternativ oder zusätzlich Gleitgel, eine andere Flüssigkeit oder Lösung zwischen den äußeren tubusförmigen Körper und die sterile Hülle angeordnet werden. Alternativ oder zusätzlich können Oberflächenbeschichtungen verwendet werden, die den Reibungswiderstand zwischen der sterilen Hülle und dem äußeren tubusförmigen Körper herabsetzen. Alternativ oder zusätzlich kann durch dichten Abschluss ein luftleerer Raum im Sinne eines Vakuums erzeugt und gehalten werden. Alternativ oder zusätzlich können Nanotechnologien Verwendung finden.

Mit einer derartigen sterilen Hülle kann ein abgeschlossenes System geschaffen werden, dass die Hygieneaspekte erfüllt. Zudem entsteht ein äußerst robustes Punktionssystem, welches auch für Punktionen unter widrigen Umständen und in besonderen Situation geeignet ist. Alternativ oder zusätzlich kann die sterile Hülle auch rohrförmig bzw. lamellenförmig ausgebildet sein. Bei Passage des Punktionssystems durch die Haut wird hierbei die sterile Hülle ähnlich einem Teleskopstab verkürzt, indem sich verschiedene feine Schichten der sterilen Hülle überlappen und die sterile Hülle somit verkürzen. Alternativ oder zusätzlich kann die sterile Hülle auch spalt- oder abreißbar und somit entfernbar sein, wenn sich das Punktionssystem in der endgültigen Position befindet und somit keine sterile Hülle mehr erforderlich ist. Die sterile Hülle kann vorteilhaft transparent gestaltet sein, so dass der Vorschiebevorgang des äußeren tubusförmigen Körpers durch die Haut des Patienten zu jedem Zeitpunkt genau verfolgt werden kann. Die sterile Hülle ist vorteilhaft schnitt- und stichfest gestaltet.

Die Erfindung kann mit folgenden Merkmalen vorteilhaft weitergebildet werden:
- Der innere tubusförmige Körper kann seitlich in das Punktionssystem einführbar sein, wobei zum Beispiel ein Verbindungselement eine Verbindung in das Lumen des äußeren tubusförmigen Körpers ermöglichen kann. Der innere tubusförmige Körper kann sich hierbei vor dem Einführen z.B. bereits in einer zusätzlichen sterilen Hülle befinden.
- Eine Verschieblichkeit der einzelnen Schichten kann zum Beispiel mit Hilfe eines Drehrades erfolgen, indem das Drehrad mit der zu verschiebenden Schicht derartig in Wechselwirkung steht, dass durch Drehen des Drehrades eine der Schichten vor und/oder zurück verschoben werden kann.
- Die Punktionsnadel ist derartig im inneren tubusförmigen Körper angeordnet, dass nach erfolgter Punktion, die Punktionsnadel durch das einströmende Fluid in den inneren tubusförmigen Körper zurück verschoben werden kann
- Die einzelnen Schichten können gerade im Bereich der Fixierung durch das Fixierelement verstärkt ausgebildet sein, insbesondere durch eine geeignete Materialauswahl, um Beschädigungen am Punktionssystem im Bereich der Fixierung zu vermeiden.
- Der äußere tubusförmige Körper kann Ausnehmungen zum Flüssigkeitsauslass oder -einlass aufweisen. Die Ausnehmungen können hierbei am patientennahen Ende des äußeren tubusförmigen Körpers positioniert werden. Dabei können mehrere Auslasslöcher angebracht sein, um die Durchflussrate der zu infundierenden Flüssigkeit zu erhöhen. Die Form der Ausnehmungen ist variabel gestaltbar, sodass die Flussrichtung günstig beeinflusst werden kann. So können die Ausnehmungen insbesondere schlitzförmig oder angeschrägt ausgebildet sein.
- Die Ausnehmungen können Rückhalteelemente aufweisen, die einen Fluss von Flüssigkeiten nur in eine definierte Richtung zulassen. Dies kann z.B. bei Dialysekathetern vorteilhaft sein, bei denen jeweils ein eigenständiger Kanal für eine Flüssigkeit nur in eine Richtung passierbar sein darf.
- Das Punktionssystem kann ein Sicherungselement haben, wobei das Sicherungselement nach Entfernen des Punktionssystems über die Punktionsnadel gelangt, sodass keine Stichverletzungen beim Patienten oder beim Anwender auftreten können. Denkbar wäre auch ein Zugmechanismus als Sicherungselement, bei dem eine Sicherung durch Umklappen der Punktionsnadel erfolgt.
- Die Applikation des Punktionssystems ist in vielen Anwendungsbereichen möglich. Hierbei kann z.B. ein Farb- und/oder Zahlenschema den Applikationsprozess verdeutlichen und damit die Patientensicherheit erheblich erhöhen.
- Das Punktionssystem kann sowohl hydrophobe als auch hydrophile Komponenten aufweisen, wobei anwendernahe Komponenten z.B. hydrophob ausgebildet sein können, damit Flüssigkeiten abgewiesen werden und somit das Punktionssystem weniger verunreinigen. Dies kann zum Beispiel im Zuge einer entsprechenden Oberflächenbeschichtung der einzelnen Schichten erfolgen. Zudem hat es den Vorteil, dass die Einführung der einzelnen Schichten in ein Körperteil erleichtert werden kann und/oder die Strömungsverhältnisse innerhalb der tubusförmigen Körper beeinflusst werden können.
- Der äußere tubusförmige Körper kann schnitt- und stichfest ausgebildet sein. Dies kann z.B. über einen Überzug oder durch die Verwendung eines entsprechenden Materials erfolgen. Es ist aber auch denkbar, dass alle Schichten des Punktionssystems schnitt- und stichfest ausgebildet sind.
- Das Punktionssystem kann ein Detektorelement haben, wobei das Detektorelement druckempfindlich ausgebildet ist und im Falle eines erhöhten Druckes, sich das Detektorelement mit der Flüssigkeit füllt. Auf diese Weise kann eine arterielle Punktion detektiert werden, da der Druck im arteriellen System grundsätzlich größer als im venösen System ist. Das Detektorelement kann dabei als drucksensitive Weiche bzw. drucksensitives Ventil ausgebildet sein, das zwei voneinander getrennte Indikatorkammern je nach den herrschenden Druckverhältnissen, zum Beispiel im Blutkreislauf eines Lebewesens, mit Blut füllt. Dadurch kann zwischen einer venösen und arteriellen Punktion unterschieden werden. Der Zustrom zur "arteriellen Kammer" öffnet sich erst, wenn ein gewisser Druck überschritten wird. Bei Füllung dieser Kammer mit Blut kann von einer arteriellen Fehlpunktion ausgegangen werden, wenn eine venöse Punktion angestrebt wird. Dies erhöht die Patientensicherheit zusätzlich. Die Drucksensitivität kann hierbei variabel eingestellt und zum Beispiel an die Kreislaufsituation bzw. die klinische Situation des Patienten angepasst werden.
- Das Punktionssystem kann Rückschlagventile haben, die ein retrogrades Herausfließen von Flüssigkeiten und ein Eindringen von Luft in das Punktionssystem und damit in den Patientenkörper verhindern.
- Der äußere tubusförmigen Körpers kann ein Ventil bzw. einen Ventilmechanismus besitzen, welcher derartig ausgebildet ist, dass bei Entfernung des inneren tubusförmigen Körpers ein automatischer Verschluss des den inneren tubusförmigen Körper aufnehmenden Arbeitslumens des äußeren tubusförmigen Körpers erfolgt.
- Das Punktionssystem kann an diversen Stellen, die Blutkontakt haben können, mit einem Material beschichtet sein, wobei das Material durch Verfärbung den Sauerstoffgehalt des Blutes bzw. den Sauerstoffpartialdruck des Blutes anzeigt.
- Das Punktionssystem kann antibakteriell bzw. antimikrobiell beschichtet sein.
- Das Punktionssystem kann an wenigstens einer Stelle mit einer speziellen Oberflächenbeschichtung versehen sein, die eine Kontamination z.B. mit Bakterien anzeigt. Dieses Oberflächenmaterial kann auch fluoreszierende Eigenschaften haben.
- Alternativ ist auch ein Spray denkbar, das nach Aufsprühen eine Kontamination sichtbar machen kann
- Zum Ablesen der Werte zum Beispiel des Sauerstoffgehalts kann eine Farbtabelle Verwendung finden, die auch direkt auf dem Punktionssystem oder der Verpackung des Punktionssystems abgedruckt werden kann
- Das Punktionssystem kann längenverstellbar ausgebildet sein, um eine individuelle Anpassung an unterschiedliche Patienten zu erreichen. Zum Beispiel kann das Punktionssystem zumindest teilweise ziehharmonikaförmig ausgebildet sein, um eine Längenverstellbarkeit zu erreichen.
- In einem oben beschriebenen Punktionssystem kann ein herkömmlicher Seldinger-Draht verwendet werden, indem dieser durch die Punktionsnadel oder den inneren oder äußeren tubusförmigen Körper in den Patienten eingeführt werden kann.
- Im Falle einer akzidentiellen Abknickung des Punktionssystems, kann es von Vorteil sein, wenn das Aspirationsmittel blockierend ausgebildet ist, sodass ein Ansaugen oder eine Zugabe von Flüssigkeiten nicht mehr möglich ist.
- Einzelne oder mehrere Komponenten des Punktionssystems können knet-oder verformbar sein, insbesondere durch aktive äußere Einflüsse. Dadurch ist eine individuelle Anpassung an den jeweiligen Patienten möglich, zum Beispiel auf die individuelle Größe eines Patienten. Zudem ist auch eine Anpassung an die jeweiligen anatomischen Besonderheiten, wie zum Beispiel spezielle Verläufe von Blutgefäßen und spezielle Konfigurationen von Körperhöhlen und Körperzwischenräumen, möglich.
- Einzelne oder mehrere Komponenten des Punktionssystems können fest bzw. starr ausgebildet sein. Insbesondere können für die Anwendung in speziellen Bereichen, wie z.B. im Bereich der minimalinvasiven Chirurgie, auch die meisten oder alle Komponenten fest bzw. starr ausgebildet sein. Hierdurch entsteht ein Punktionssystem, das Trokar-ähnliche Eigenschaften hat und in seiner endgültigen Position nach Entfernen einer oder mehrerer Schichten wie des inneren tubusförmigen Körpers, beispielhaft chirurgischen oder endoskopischen Instrumenten den Weg in eine Körperhöhle oder einen Körperzwischenraum erschließen kann.

Die Erfindung wird nachfolgend anhand von Ausführungsbeispielen unter Verwendung von Zeichnungen näher erläutert. Es zeigt:
- Figur 1: - Ein Punktionssystem in einer Schnittansicht;
- Figur 2: - Ein Punktionssystem gemäß Figur 1 in einer in einer um 90° gedrehten Position in einer Seitenansicht;
- Figur 3: - Eine vergrößerte Darstellung des patientennahen Endes des Punktionssystems in einer Seitenansicht;
- Figur 4: - Eine vergrößerte Darstellung des patientennahen Endes des Punktionssystems gemäß Figur 3 in einer Schnittansicht;
- Figur 5: - Eine vergrößerte Darstellung des patientenfernen Endes des Punktionssystems gemäß Figur 1 in einer Schnittansicht.

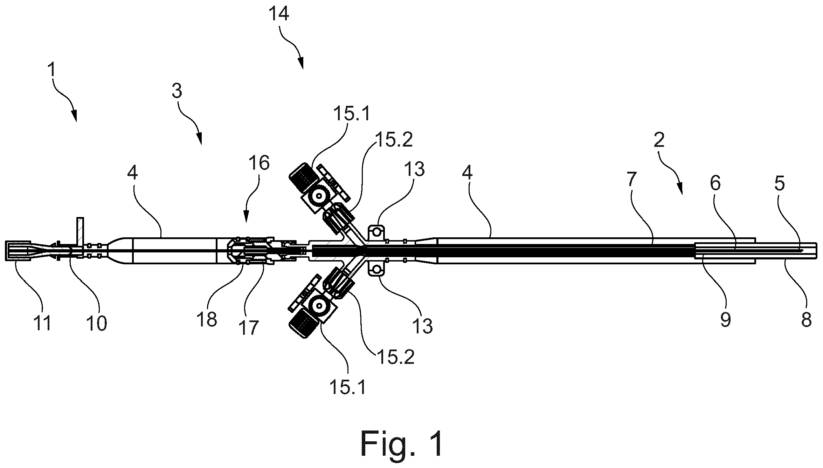

Figur 1 zeigt ein Punktionssystem 1 in einer Schnittansicht. Alle Lumen des Punktionssystems 1 können im Auslieferungszustand mit sterilem Kochsalz oder destilliertem Wasser gefüllt sein, damit das Punktionssystem 1 in einem entlüftetem Zustand direkt verwendet werden kann.

Das Punktionssystem 1 hat ein patientennahes Ende 2, wobei das patientennahe Ende zur Einführung in einen Patienten, zum Beispiel in eine Vene, eingerichtet ist. Ein patientenfernes Ende 3 ist vom Patienten abgewandt angeordnet, wobei eine Bedienung durch einen Anwender des Punktionssystems 1 über das patientenferne Ende 3 erfolgt. Es wird deutlich, dass das Punktionssystem 1 eine äußere Hülle 4, insbesondere eine sterile Hülle 4, hat, wobei eine Punktionsnadel 5, ein innerer tubusförmiger Körper 6 und ein äußerer tubusförmiger Körper 7 innerhalb der sterilen Hülle angeordnet sind. Innerhalb des Arbeitslumen des inneren tubusförmigen Körpers 6 ist die Punktionsnadel 5 längsverschieblich geführt, wobei der innere tubusförmige Körper 6 im Arbeitslumen des äußeren tubusförmigen Körpers 7 längsverschieblich geführt ist.

Figur 1 zeigt das Punktionssystem 1 in einem Auslieferungszustand. Im Auslieferungszustand ragt die Punktionsnadel 5 am patientennahen Ende 2 noch nicht aus dem Punktionssystem 1 heraus. Die Punktionsnadel 5 ist noch mit einer Schutzkappe 8 versehen, die vor Applikation an einem Patienten erst entfernt werden muss. Eine derartige Schutzkappe 8 verhindert, dass die Punktionsnadel 5 den Anwender und/oder den Patienten bereits vor der Applikation verletzt und die Punktionsnadel unsterilen Einflüssen ausgesetzt ist.

Die Applikation des Punktionssystems 1 an einem Patienten kann zum Beispiel in folgenden Schritten ablaufen, die an dem Punktionssystem 1 durch eine entsprechende Farbkodierung oder Nummerierung dargestellt sein können:
1. Punktion einer Vene mit der Punktionsnadel 5
2. Vorschieben des inneren tubusförmigen Körpers 6 in die Vene an die gewünschte Position
3. Entfernen/Zurückschieben der Punktionsnadel 5
4. Verschließen des inneren tubusförmigen Körpers 6
5. Vorschieben des äußeren tubusförmigen Körpers 7 über den inneren tubusförmigen Köper 6 in die Vene
6. Entfernen/Zurückschieben des inneren tubusförmigen Körpers 6

Der äußere tubusförmige Körpers 7 hat einen Dilatationskörper 9, wobei durch den Dilatationskörper 9 eine schrittweise Aufweitung der punktierten Stelle erfolgt, sodass der äußere tubusförmige Körper 7 aufgrund des größeren Durchmessers als der innere tubusförmige Körper 6 in das punktierte Körperteil eingeführt werden kann. Der Dilatationskörper 9 läuft dabei in einem Winkel von weniger als 11 Grad zu, vorzugsweise von weniger als 10,5°, sodass eine sichere Aufweitung der punktierten Stelle gewährleistet ist.

Es wird deutlich, dass die sterile Hülle 4 stabilisierend auf das Punktionssystem 1 wirkt. Die sterile Hülle 4 kann die punktierte Stelle aufgrund seiner Maße nicht passieren. So kann die sterile Hülle 4 beispielsweise aus einem festen Material gebildet sein, wobei das Punktionssystem 1 mit dem inneren tubusförmigen Körper 6 und dem äußeren tubusförmigen Körper 7 innerhalb des Körpers eines Patienten flexible Eigenschaften hat und durch die äußere Hülle 4 außerhalb des Körpers stabilisierend auf das Punktionssystem 1 wirkt, sodass die Applikation am Patienten erleichtert ist. Die Hülle 4 ist dabei transparent ausgebildet, damit zu jedem Zeitpunkt die Längsverschiebung der Punktionsnadel 5, des inneren tubusförmigen Körpers 6 und des äußeren tubusförmigen Körpers 7 für den Anwender ersichtlich ist. Die Punktionsnadel 5, der innere tubusförmige Körper 6 und der äu-ßere tubusförmige Körper 7 können dabei transparent ausgebildet sein.

Am patientenfernen Ende 3 ist ein Anschlusskörper 10 zum Anschluss einer Spritze vorgesehen, wobei die Spritze zum Beispiel zum Durchführen einer Blutaspiration genutzt werden kann. Eine Mandrin 11 am Anschlusskörper 10 erleichtert die Einführung des äußeren und inneren tubusförmigen Körpers 7, 6 und der Punktionsnadel 5.

Das Punktionssystem 1 hat weiterhin zwei Befestigungselemente 13, wobei die Befestigungselemente 13 dazu eingerichtet sind, das Punktionssystem 1 am Patienten zu fixieren. Dazu kann das Punktionssystem 1 zum Beispiel an den Befestigungselementen 13 an dem Patienten festgenäht werden.

An dem Punktionssystem 1 ist ein Y-Stück 14 zwischen dem patientennahen Ende 2 und dem patientenfernen Ende 3 angeordnet. An dem Y-Stück 14 sind Dreiwegehähne 15.1 angeschlossen. So können auf einfache Weise mehrere Zugänge zum Punktionssystem bereitgestellt werden, die zum Beispiel eine gleichzeitige Verabreichung von unterschiedlichen Flüssigkeiten ermöglichen. Die Dreiwegehähne 15.1 sind dabei über ein Rastmechanismus 15.2 mit dem Y-Stück 14 verbunden, der eine schnelle und zuverlässige Verbindung durch Einrasten der Dreiwegehähne 15.1 in die Rastmechanismen 15.2 ermöglicht. Über die Dreiwegehähne 15.1 können zum Beispiel Spritzen, Infusionen oder eine elektrische Anschlussleitung für eine kontinuierliche EKG Ableitung über das Punktionssystem 1 angeschlossen werden. Vorteilhafterweise können auch Filter in den Dreiwegehähnen integriert sein, die zum Beispiel das Eindringen von Luft in den menschlichen Körper verhindern können. Dabei können Ventile in den Dreiwegehähnen integriert sein, die den Fluss von Flüssigkeiten lediglich in eine definierte Richtung zulassen. Auf diese Weise kann ein unbeabsichtigter Flüssigkeitsfluss aus dem Punktionssystem in Richtung des Anwenders verhindert werden.

Das Punktionssystem 1 hat ein Fixierelement 16, wobei das Fixierelement 16 aus einer längsgeschlitzten Hülse 17 und einer Überwurfmutter 18 gebildet ist. Es wird deutlich, dass durch das Fixierelement 16 die Längsverschieblichkeit der Punktionsnadel 5, des inneren tubusförmigen Körpers 6 und/oder des äußeren tubusförmigen Körpers 7 vermindert oder aufgehoben werden kann. Durch Anziehen der Überwurfmutter 18 können die Punktionsnadel 5, der inneren tubusförmigen Körpers 6 und/oder des äußeren tubusförmigen Körpers 7 gegeneinander so festgestellt werden, dass das Punktionssystem 1 in seiner Lage stabilisiert und somit die Applikation am Patienten erleichtert wird. Es ist zum Beispiel auch denkbar, dass nur zwei der drei Schichten gegeneinander längsverschieblich fixiert werden können, sodass je nach Applikationsschritt eine Fixierung anderer Schichten erfolgen kann.

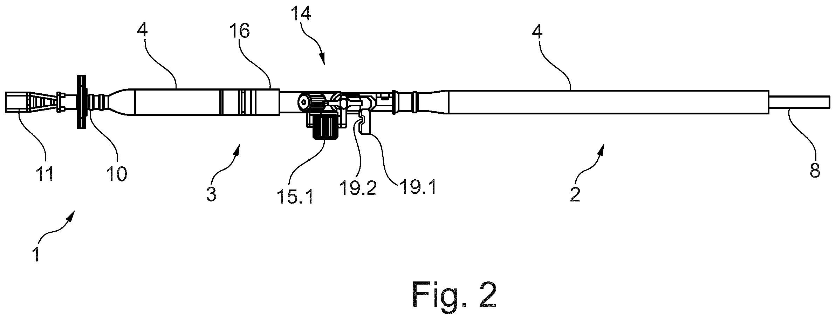

Die Figur 2 zeigt ein Punktionssystem 1 gemäß Figur 1 in einer um 90° gedrehten Position in einer Seitenansicht. In dieser Ansicht ist zu erkennen ist, dass das Punktionssystem 1 einen Haltekörper 19.1 hat, wobei der Haltekörper 19.1 die Anwendung für den Bediener erleichtert, indem es dem Anwender ermöglicht, beim Applikationsvorgang mit der zweiten Hand das patientennahe Ende 2 zu halten und zu steuern, bzw. weiter zu stabilisieren.

Der Haltekörper hat dabei eine Ausnehmung 19.2, die zum Führen eines Fadens eingerichtet ist. Durch eine Naht, die über die Ausnehmung 19.2 und den Befestigungselemente 13 (siehe Figur 1) am Patienten festgenäht ist, kann so eine Zugkraft in Richtung des Patienten ausgeübt werden, sodass die Fixierung am Patienten verbessert ist.

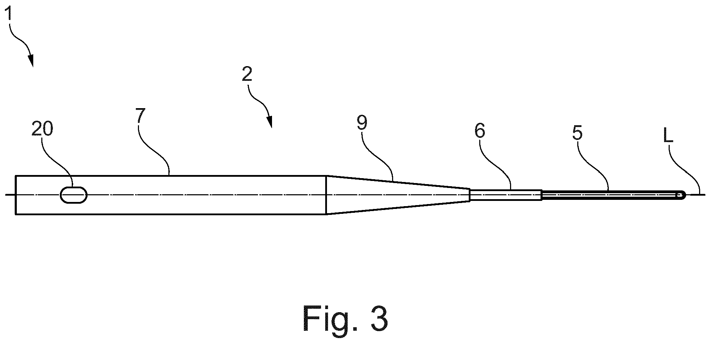

Figur 3 zeigt eine vergrößerte Darstellung des patientennahen Endes 2 des Punktionssystems 1. Deutlich wird der dreischichte Aufbau des Punktionssystems 2. Dabei ist die Punktionsnadel 5 innerhalb eines Arbeitslumens des inneren tubusförmigen Körpers 6 längsverschieblich in Richtung der Längssachse L und der innere tubusförmige Körper 6 in einem Arbeitslumen des äußeren tubusförmigen Körpers 7 angeordnet. Deutlich wird weiterhin, dass der äußere tubusförmige Körper 7 ein Dilatationselement 9 hat, wobei das Dilatationselement 9 in einem spitzen Winkel von weniger als 11° zuläuft, damit eine Aufweitung des punktierten Körperteils erreicht werden kann, sodass der äußere tubusförmige Körper 7 durch die punktierte Stelle in den Körper des Patienten geführt werden kann. Ein Dilatationskörper am inneren tubusförmigen Körper 6 ist hingegen nicht notwendig, da der Durchmesserunterschied zur Punktionsnadel 5 so gering ist, dass der innere tubusförmige Körper 6 durch die punktierte Stelle unproblematisch in den Körper eingeführt werden kann.

Es ist weiterhin erkennbar, dass der äußere tubusförmige Körper 7 ein Loch 20 aufweist. Ein derartiges Loch 20 an der Außenseite des äußeren tubusförmigen Körpers 7 kann als Flüssigkeitsauslass dienen. Das Loch 20 ist dabei nicht in seiner Form beschränkt, sondern kann vielmehr auch anders ausgestaltet sein. Alternativ können die Löcher zum Beispiel angeschrägt oder schlitzförmig ausgebildet sein, um die Flussrichtung von Flüssigkeiten günstig zu beeinflussen.

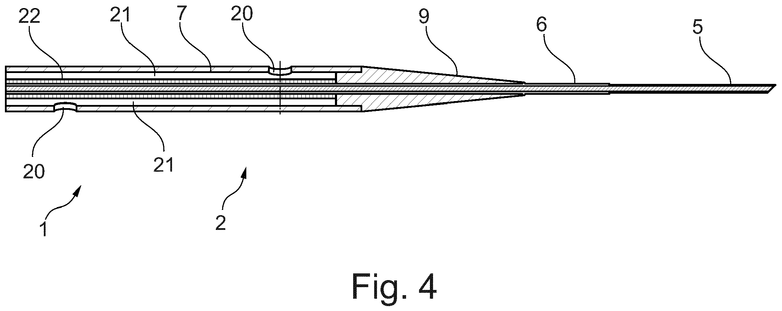

Figur 4 zeigt weiterhin eine vergrößerte Darstellung des patientennahen Endes 2 des Punktionssystems 1 gemäß Figur 3 in einer Schnittansicht. Erkennbar ist, dass das Punktionssystem 1 an der Außenseite des äußeren tubusförmigen Köpers 7 mehrere Löcher 20 hat, wobei die Löcher 20 an unterschiedlichen Positionen am patientennahen Ende 2 angeordnet sind.

Den Löchern 20 ist jeweils ein Kanal 21 des äußeren tubusförmigen Körpers zugeordnet. Dies kann besonders von Vorteil sein, wenn sowohl der Einlass als auch der Auslass von Flüssigkeiten möglich sein soll. Dies kann dann über einen der autarken Kanäle 21 erfolgen.

Aus der Figur 4 ist weiterhin ersichtlich, dass der innere tubusförmige Körper 6 ein über den Umfang verlaufendes Dichtelement 22 hat. Das Dichtelement 22 kann zum Beispiel eine PTFE-Beschichtung sein und an der Außenseite des inneren tubusförmigen Körpers 6 aufgebracht sein. Hat der innere tubusförmige Körper 6 beispielsweise eine wickelförmige Struktur, ist eine Abdichtung notwendig, sodass kein Fluidstrom zwischen dem inneren tubusförmigen Körper 6 und dem äußeren tubusförmigen Körper 7 auftreten kann. In Figur 4 verläuft das Dichtelement 22 nur teilweise um den tubusförmigen Körper 6. Es ist aber auch denkbar, dass das Dichtelement 22 um den gesamten Umfang des tubusförmigen Körpers 6 verlaufen kann.

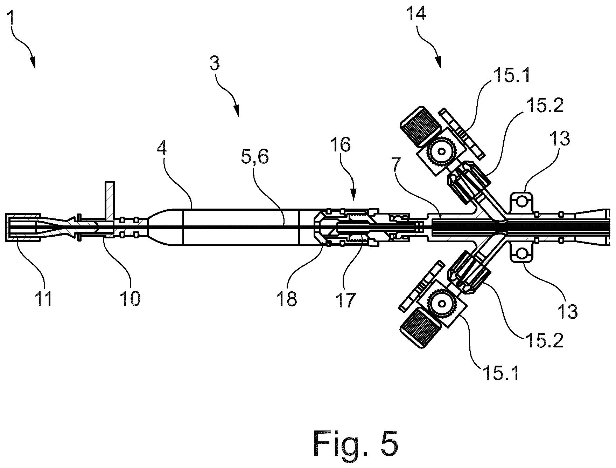

Figur 5 zeigt eine vergrößerte Darstellung des patientenfernen Endes 3 des Punktionssystems 1 gemäß Figur 1 in einer Schnittansicht. Das Fixierelement 16 ist aus einer längsgeschlitzten Hülse 17 und einer Überwurfmutter 18 gebildet. Es wird deutlich, dass nach Anziehen der Überwurfmutter 18 die Punktionsnadel 5 und der innere tubusförmige Körper 6 in die längsgeschlitzte Hülse 17 gedrückt und somit die Längsverschieblichkeit verringert wird. Bei welchen Schichten die Längsverschieblichkeit verringert wird ist dabei nicht von diesem Ausführungsbeispiel abhängig. Denkbar wäre auch, dass der äußere tubusförmige Körper 7 in der längsgeschlitzten Hülse 17 aufgenommen wird, sodass die Längsverschieblichkeit aller drei Schichten verringert oder aufgehoben werden kann.

Die Figuren 1 bis 5 verstehen sich als mögliche Ausführungsbeispiele. Andere Formen der erfindungsgemäßen Lehre sind weiterhin denkbar. Des Weiteren sind die Ausgestaltungen der Ausführungsbeispiele nicht untrennbar miteinander verknüpft, sodass z.B. die Ausführung der Erfindung nicht abhängig von den speziell beschriebenen Ausgestaltungen der Ausführungsbeispiele ist. So ist eine Variabilität zum Beispiel von der Anzahl, Länge oder Größe der einzelnen Elemente jederzeit denkbar.

## Patentansprüche

1. Punktionssystem (1) mit einem äußeren tubusförmigen Körper (7), der zum Verweiten in einem Körperteil eines Lebewesens eingerichtet ist, wobei das Punktionssystem wenigstens einen inneren tubusförmigen Körper (6) und eine Punktionsnadel (5) aufweist, wobei der innere tubusförmige Körper durch ein Arbeitslumen des äußeren tubusförmigen Körpers geführt ist und gegenüber dem äu-ßeren tubusförmigen Körper längsverschieblich ist, und die Punktionsnadel durch ein Punktionslumen des inneren tubusförmigen Körpers geführt ist und der innere tubusförmige Körper gegenüber der Punktionsnadel längsverschieblich ist, wobei das Punktionssystem ein manuell betätigbares Fixierelement (16) aufweist, das durch manuelle Betätigung zumindest in eine Fixierstellung und in eine Lösestellung stellbar ist, wobei in der Fixierstellung die Längsverschieblichkeit
a1) des inneren tubusförmigen Körpers gegenüber dem äußeren tubusförmigen Körper und/oder
a2) der Punktionsnadel gegenüber dem inneren tubusförmigen Körper und/oder
a3) der Punktionsnadel gegenüber dem äußeren tubusförmigen Körper aufgehoben oder vermindert ist,
**dadurch gekennzeichnet, dass** das Fixierelement durch manuelle Betätigung in unterschiedliche Fixierstellungen stellbar ist, in denen die Längsverschieblichkeit jeweils hinsichtlich unterschiedlicher Kombinationen der Merkmale Längsverschieblichkeit
- des inneren tubusförmigen Körpers gegenüber dem äußeren tubusförmigen Körper,
- der Punktionsnadel gegenüber dem inneren tubusförmigen Körper,
- der Punktionsnadel gegenüber dem äußeren tubusförmigen Körper einstellbar ist.

2. Punktionssystem nach Anspruch 1, **dadurch gekennzeichnet, dass** mittels des Fixierelements in der Fixierstellung die Längsverschieblichkeit auf ein durch die manuelle Betätigung einstellbares Maß verminderbar ist.

3. Punktionssystem nach einem der vorhergehenden Ansprüche, **dadurch gekennzeichnet, dass** das Fixierelement durch manuelle Betätigung in unterschiedliche Fixierstellungen stellbar ist, in denen die Längsverschieblichkeit auf jeweilige voneinander unterschiedliche Maße verminderbar ist.

4. Punktionssystem nach einem der vorhergehenden Ansprüche, **dadurch gekennzeichnet, dass** das Fixierelement als Klemmelement ausgebildet ist oder ein Klemmelement aufweist, wobei mittels des Klemmelements
a1) der innere tubusförmige Körpers gegenüber dem äußeren tubusförmigen Körper und/oder
a2) die Punktionsnadel gegenüber dem inneren tubusförmigen Körper und/oder
a3) die Punktionsnadel gegenüber dem äußeren tubusförmigen Körper festklemmbar ist.

5. Punktionssystem nach einem der vorhergehenden Ansprüche, **dadurch gekennzeichnet, dass** das Punktionssystem ein Dichtelement (22) aufweist, durch das der innere tubusförmige Körper gegenüber dem äußeren tubusförmigen Körper zumindest am patientennahen Ende (2) abgedichtet ist.

6. Punktionssystem nach Anspruch 5, **dadurch gekennzeichnet, dass** das Dichtelement an der Außenseite des inneren tubusförmigen Körpers fixiert ist.

7. Punktionssystem nach Anspruch 5 oder 6, **dadurch gekennzeichnet, dass** das Dichtelement als Kunststoffbeschichtung des inneren tubusförmigen Körpers ausgebildet ist, insbesondere als PTFE-Beschichtung.

8. Punktionssystem nach einem der vorhergehenden Ansprüche, **dadurch gekennzeichnet, dass** der innere tubusförmige Körper an der Außenseite eine unebene Oberfläche aufweist.

9. Punktionssystem nach Anspruch 8, **dadurch gekennzeichnet, dass** die unebene Oberfläche zumindest in einem Teilbereich des patientennahen Endes durch das Dichtelement überdeckt ist, insbesondere luftdicht überdeckt ist.

10. Punktionssystem nach einem der vorhergehenden Ansprüche, **dadurch gekennzeichnet, dass** das Punktionssystem wenigstens ein Befestigungselement (13) aufweist, wobei das Befestigungselement zum Fixieren des Punktionssystems an einem Patienten ausgebildet ist.

11. Punktionssystem nach einem der vorhergehenden Ansprüche, **dadurch gekennzeichnet, dass** das Punktionssystem einen Haltekörper (19.1) zum erleichterten Halten des Punktionssystems durch den Anwender aufweist.

12. Punktionssystem nach Anspruch 11, **dadurch gekennzeichnet, dass** der Haltekörper eine Ausnehmung (19.2) hat, wobei die Ausnehmung zum Führen eines Fadens eingerichtet ist.

13. Punktionssystem nach einem der vorhergehenden Ansprüche, **dadurch gekennzeichnet, dass** das Punktionssystem einen Mandrin (11) zur Stabilisierung des inneren tubusförmigen Körpers, des äußeren tubusförmigen Körpers und/oder der Punktionsnadel hat.

14. Punktionssystem nach einem der vorhergehenden Ansprüche, **dadurch gekennzeichnet, dass** der innere tubusförmige Körper zumindest teilweise aus einem Metall besteht oder mit einem Metall beschichtet ist.

15. Punktionssystem nach einem der vorhergehenden Ansprüche, **dadurch gekennzeichnet, dass** das Punktionssystem am patientennahen Ende einen zum Einführen des äußeren tubusförmigen Körpers in das Körperteil dienenden Dilatationskörper (9) aufweist, durch den der innere tubusförmige Körper hindurch geführt ist, wobei der Dilatationskörper in einem spitzen Winkel von weniger als 11 Grad zuläuft.

## Claims

1. Puncture system (1) comprising an outer tubular body (7) adapted to dwell in a body part of a living being, said puncture system comprising at least an inner tubular body (6) and a puncture needle (5), wherein the inner tubular body is guided through a working lumen of the outer tubular body and is longitudinally displaceable relative to the outer tubular body, and the puncture needle is guided through a puncture lumen of the inner tubular body and the inner tubular body is longitudinally displaceable relative to the puncture needle, wherein the puncture system has a manually actuable fixing element (16) which can be set by manual actuation at least into a fixing position and into a release position, wherein in the fixing position the longitudinal displaceability
a1) of the inner tubular body relative to the outer tubular body and/or
a2) of the puncture needle relative to the inner tubular body and/or
a3) of the puncture needle relative to the outer tubular body is eliminated or reduced,
**characterized in that** the fixing element can be set by manual actuation into different fixing positions in which the longitudinal displaceability can be adjusted in each case with respect to different combinations of the features longitudinal displaceability
- of the inner tubular body relative to the outer tubular body,
- of the puncture needle relative to the inner tubular body,
- of the puncture needle relative to the outer tubular body.

2. Puncture system according to claim 1, **characterized in that** by means of the fixing element in the fixing position the longitudinal displaceability can be reduced to a degree adjustable by manual actuation.

3. Puncture system according to one of the preceding claims, **characterized in that** the fixing element is adjustable by manual actuation into different fixing positions in which the longitudinal displaceability can be reduced to respective mutually different degrees.

4. Puncture system according to one of the preceding claims, **characterized in that** the fixing element is designed as a clamping element or has a clamping element, wherein by means of the clamping element
a1) the inner tubular body relative to the outer tubular body and/or
a2) the puncture needle relative to the inner tubular body and/or
a3) the puncture needle relative to the outer tubular body can be clamped.

5. Puncture system according to one of the preceding claims, **characterized in that** the puncture system comprises a sealing element (22) by which the inner tubular body is sealed off from the outer tubular body at least at the end (2) near the patient.

6. Puncture system according to claim 5, **characterized in that** the sealing element is fixed to the outside of the inner tubular body.

7. Puncture system according to claim 5 or 6, **characterized in that** the sealing element is formed as a plastic coating of the inner tubular body, in particular as a PTFE coating.

8. Puncture system according to any one of the preceding claims, **characterized in that** the inner tubular body has an uneven surface on the outside.

9. Puncture system according to claim 8, **characterized in that** the uneven surface is covered, in particular covered in an airtight manner, by the sealing element at least in a partial region of the end near the patient.

10. Puncture system according to any one of the preceding claims, **characterized in that** the puncture system comprises at least one fastening element (13), the fastening element being designed for fixing the puncture system to a patient.

11. Puncture system according to one of the preceding claims, **characterized in that** the puncture system has a holding body (19.1) for facilitating holding of the puncture system by the user.

12. Puncture system according to claim 11, **characterized in that** the holding body has a recess (19.2), the recess being arranged for guiding a thread.

13. Puncture system according to one of the preceding claims, **characterized in that** the puncture system has a stylet (11) for stabilizing the inner tubular body, the outer tubular body and/or the puncture needle.

14. Puncture system according to any one of the preceding claims, **characterized in that** the inner tubular body is at least partially made of a metal or is coated with a metal.

15. Puncture system according to one of the preceding claims, **characterized in that** the puncture system comprises at the end near the patient a dilatation body (9) serving to introduce the outer tubular body into the body part, through which the inner tubular body is passed, the dilatation body tapering at an acute angle of less than 11 degrees.

## Revendications

1. Système de ponction (1) comprenant un corps tubulaire extérieur (7) conçu pour rester dans une partie du corps d'un être vivant, le système de ponction présentant au moins un corps tubulaire intérieur (6) et une aiguille de ponction (5), le corps tubulaire intérieur étant mené à travers une lumière de travail du corps tubulaire extérieur et étant déplaçable longitudinalement par rapport au corps tubulaire extérieur, et l'aiguille de ponction étant menée à travers une lumière de ponction du corps tubulaire intérieur, et le corps tubulaire intérieur étant déplaçable longitudinalement par rapport à l'aiguille de ponction,
dans lequel
le système de ponction présente un élément de fixation (16) susceptible d'être actionné manuellement, qui peut être placé par actionnement manuel au moins dans une position de fixation et dans une position de libération,
dans la position de fixation, la mobilité longitudinale
a1) du corps tubulaire intérieur par rapport au corps tubulaire extérieur et/ou
a2) de l'aiguille de ponction par rapport au corps tubulaire intérieur et/ou
a3) de l'aiguille de ponction par rapport au corps tubulaire extérieur est supprimée ou réduite,
**caractérisé en ce que**
l'élément de fixation peut être placé, par actionnement manuel, dans différentes positions de fixation dans lesquelles la mobilité longitudinale est réglable en ce qui concerne différentes combinaisons des caractéristiques de la mobilité longitudinale
- du corps tubulaire intérieur par rapport au corps tubulaire extérieur et/ou
- de l'aiguille de ponction par rapport au corps tubulaire intérieur et/ou
- de l'aiguille de ponction par rapport au corps tubulaire extérieur.

2. Système de ponction selon la revendication 1,
**caractérisé en ce qu'**au moyen de l'élément de fixation dans la position de fixation, la mobilité longitudinale peut être réduite à un degré réglable par l'actionnement manuel.

3. Système de ponction selon l'une des revendications précédentes,
**caractérisé en ce que** l'élément de fixation peut être placé, par actionnement manuel, dans différentes positions de fixation dans lesquelles la mobilité longitudinale peut être réduite à des degrés respectifs différents les uns des autres.

4. Système de ponction selon l'une des revendications précédentes, **caractérisé en ce que** l'élément de fixation est réalisé sous forme d'élément de serrage ou présente un élément de serrage, l'élément de serrage permettant de serrer
a1) le corps tubulaire intérieur par rapport au corps tubulaire extérieur et/ou
a2) l'aiguille de ponction par rapport au corps tubulaire intérieur et/ou
a3) l'aiguille de ponction par rapport au corps tubulaire extérieur.

5. Système de ponction selon l'une des revendications précédentes,
**caractérisé en ce que** le système de ponction présente un élément d'étanchéité (22) par lequel le corps tubulaire intérieur est rendu étanche par rapport au corps tubulaire extérieur au moins à l'extrémité (2) proche du patient.

6. Système de ponction selon la revendication 5,
**caractérisé en ce que** l'élément d'étanchéité est fixé sur la face extérieure du corps tubulaire intérieur.

7. Système de ponction selon la revendication 5 ou 6,
**caractérisé en ce que** l'élément d'étanchéité est réalisé sous forme de revêtement en matière plastique du corps tubulaire intérieur, en particulier sous forme de revêtement PTFE.

8. Système de ponction selon l'une des revendications précédentes,
**caractérisé en ce que** le corps tubulaire intérieur présente une surface irrégulière sur la face extérieure.

9. Système de ponction selon la revendication 8,
**caractérisé en ce que** la surface irrégulière est recouverte, en particulier de manière étanche à l'air, par l'élément d'étanchéité au moins dans une zone partielle de l'extrémité proche du patient.

10. Système de ponction selon l'une des revendications précédentes,
**caractérisé en ce que** le système de ponction présente au moins un élément d'immobilisation (13), l'élément d'immobilisation étant conçu pour fixer le système de ponction sur un patient.

11. Système de ponction selon l'une des revendications précédentes,
**caractérisé en ce que** le système de ponction présente un corps de retenue (19.1) pour faciliter à l'utilisateur de tenir le système de ponction.

12. Système de ponction selon la revendication 11,
**caractérisé en ce que** le corps de retenue présente un évidement (19.2), l'évidement étant conçu pour guider un fil.

13. Système de ponction selon l'une des revendications précédentes,
**caractérisé en ce que** le système de ponction comporte un mandrin (11) pour stabiliser le corps tubulaire intérieur, le corps tubulaire extérieur et/ou l'aiguille de ponction.

14. Système de ponction selon l'une des revendications précédentes,
**caractérisé en ce que** le corps tubulaire intérieur consiste au moins partiellement en un métal ou est revêtu d'un métal.

15. Système de ponction selon l'une des revendications précédentes,
**caractérisé en ce que** le système de ponction présente, à l'extrémité proche du patient, un corps de dilatation (9) qui sert à introduire le corps tubulaire extérieur dans la partie du corps et qui est traversé par le corps tubulaire intérieur, le corps de dilatation se terminant en un angle aigu de moins de 11 degrés.
